Europäisches Patentamt

European Patent Office

Office européen des brevets

(19)

(11) Veröffentlichungsnummer: **0 305 925 B1**

(12) # EUROPÄISCHE PATENTSCHRIFT

(45) Veröffentlichungstag der Patentschrift: **29.01.92**

(51) Int. Cl.⁵: **C07C 305/02**, C07C 303/00

(21) Anmeldenummer: **88113964.6**

(22) Anmeldetag: **26.08.88**

(54) **Verfahren zur Herstellung von Umsetzungsprodukten epoxidierter Ricinolsäureglyceride mit Schwefeltrioxid.**

(30) Priorität: **03.09.87 DE 3729484**

(43) Veröffentlichungstag der Anmeldung:
**08.03.89 Patentblatt 89/10**

(45) Bekanntmachung des Hinweises auf die
Patenterteilung:
**29.01.92 Patentblatt 92/05**

(84) Benannte Vertragsstaaten:
**DE**

(56) Entgegenhaltungen:
EP-A- 0 178 557     EP-A- 0 222 237
EP-A- 0 241 839     DE-A- 1 443 989
DE-A- 2 040 503     DE-A- 3 617 657

(73) Patentinhaber: **Henkel Kommanditgesellschaft auf Aktien
Postfach 1100 Henkelstrasse 67
W-4000 Düsseldorf-Holthausen(DE)**

(72) Erfinder: **Fabry, Bernd, Dr.
Danziger Strasse 31
W-4051 Korschenbroich(DE)**
Erfinder: **Piorr, Robert, Dr.
Kieselei 12
W-4030 Ratingen-Hösel(DE)**
Erfinder: **Clasen, Frank
Am Bandsbusch 46
W-4010 Hilden(DE)**
Erfinder: **Ritterbex, Horst
Bredstrasse 44
W-4000 Düsseldorf(DE)**
Erfinder: **Bornmann, Hans
Am Falder 22
W-4000 Düsseldorf 13(DE)**

EP 0 305 925 B1

## Beschreibung

Die Erfindung betrifft ein Verfahren zur Herstellung von Umsetzungsprodukten epoxidierter Ricinolsäureglyceride mit Schwefeltrioxid. Die erfindungsgemäß hergestellten Produkte weisen grenzflächenaktive Eigenschaften auf und können als Emulgatoren und Waschrohstoffe eingesetzt werden.

Man kann funktionelle Gruppen in das Molekül ungesättigter Fettsäureester einführen, indem man die olefinischen Doppelbindungen dieser Verbindungen in üblicher Weise durch Epoxidation in einen Oxiranring überführt und diesen mit Nucleophilen unter Bildung von 2-Hydroxyalkylderivaten öffnet.

Es ist weiterhin bekannt, Epoxide durch Umsetzung mit Schwefeltrioxid in 1,2-Glycolsulfate zu überführen. Diese Umsetzung verläuft jedoch beispielsweise bei Ethylenoxid gemäß J. Org. Chem. 25, 864 (1960), so heftig, daß die direkte Umsetzung mit Schwefeltrioxid unter Zersetzung erfolgt und nur die Umsetzung mit einer Schwefeltrioxid-Dioxan-Additionsverbindung zum gewünschten Glycolsulfat führt. Gemäß der DE-AS 20 40 503 wird daher das Epoxid zunächst mit Schwefeldioxid zum Glycolsulfit und anschließend mit Schwefeltrioxid zum Glycolsulfat umgesetzt.

Ein wichtiger Vertreter der vorstehend genannten Umsetzungsprodukte von Epoxyfettsäureestern mit Schwefeltrioxid ist der aus epoxidiertem Ricinusöl erhaltene, der z.B. als Lackverdikkungsmittel eingesetzt wird. Die Herstellung dieser Verbindung erfolgt über eine Sulfatierung von epoxidiertem Ricinusöl mit Oleum. Bei diesem Verfahren fällt jedoch eine hohe Salzbelastung an, so daß es wegen der damit verbundenen Abwasserbelastung als nachteilig anzusehen ist. Zudem weisen die Verfahrensprodukte erhebliche Sulfatkonzentrationen auf, die sich hinsichtlich des Korrosionsverhaltens nachteilig auswirken. Ähnliche Nachteile weisen Sulfierverfahren auf, die mit Schwefelsäure oder Chlorsulfonsäure arbeiten und - ebenso wie die bereits erwähnte Oleumsulfierung - in Kirk-Othmer, Encyclopedia of Chemical Technology, 2. Auflage, Band 19, Seiten 301 bis 306, J. Wiley & Sons, New York, 1983, und Winnacker-Küchler, Chemische Technologie, 4. Auflage, Band 4, Seiten 464 bis 468, Carl Hanser Verlag, München, 1984, beschrieben sind.

Demgemäß ist die Erfindung auf ein Verfahren der eingangs genannten Art gerichtet, durch das die Salzbelastung von Abwässern und Endprodukten vermieden wird. Die Lösung dieser Aufgabe beruht auf der Erkenntnis, daß die direkte Umsetzung von Epoxyfettsäureestern mit Schwefeltrioxid überraschend glatt unter technisch leicht zu steuernden Bedingungen ohne Zersetzungserscheinungen abläuft. Dabei reagiert Schwefeltrioxid zunächst sehr schnell mit den vorhandenen Epoxygruppen zu 1,2-Glycolsulfatgruppen und mit geringerer Reaktionsgeschwindigkeit mit den in den Ricinolresten vorhandenen Hydroxylgruppen zu Ricinolsulfatgruppen, die jeweils weiteren Umsetzungen mit Nucleophilen unter Bildung von Produkten mit grenzflächenaktiven Eigenschaften zugänglich sind.

Das Verfahren der Erfindung ist dadurch gekennzeichnet, daß man die epoxidierten Ricinolsäureglyceride mit einem trockenen, mit einem Trägergas verdünnten Strom gasförmigen Schwefeltrioxids und das sulfierte Reaktionsprodukt mit Nucleophilen umsetzt. Die bisher zwangsläufig gegebene Salzbelastung entfällt, da die Notwendigkeit zur Neutralisation überschüssiger Schwefelsäure entfällt.

Bei dem Verfahren der Erfindung geht man von technischen epoxidierten Ricinolsäureglyceriden aus, wie sie beispielsweise durch Epoxidierung von Ricinusöl erhalten werden können, dessen Fettsäurekomponente noch gewisse Anteile an gesättigten und/oder ungesättigten Fettsäuren enthält. Bevorzugt setzt man teilepoxidiertes Ricinusöl ein, bei dem 10 bis 30, insbesondere 10 bis 15 % der ursprünglich vorhandenen olefinischen Doppelbindungen epoxidiert sind. Im übrigen können auch Partialglyceride der epoxidierten Ricinolsäure umgesetzt werden.

Gemäß einer weiteren vorteilhaften Ausführungsform der Erfindung führt man die Sulfierung mit Luft oder Stickstoff als Trägergas durch, wobei der Trägergasstrom einen Schwefeltrioxidgehalt von 2 bis 10, insbesondere 5 bis 8 Vol.-% aufweist. Die Reaktionstemperatur bei der Sulfierung beträgt vorzugsweise 30 bis 80, insbesondere 40 bis 50 °C. Bei Temperaturen unterhalb von 30 °C lassen sich die epoxidierten Ricinolsäureglyceride nur schwer sulfatieren; bei Temperaturen oberhalb von 80 °C kommt es dagegen zu unerwünschten Nebenreaktionen, z.B. Esterspaltungen, Verkokung und deutlichen Farbverschlechterungen des Produktes.

Gemäß einer weiteren vorteilhaften Ausführungsform der Erfindung setzt man pro Mol vorhandenem Ricinolsäurerest 0,25 - 1 Mol Schwefeltrioxid ein. Beim Arbeiten in Abwesenheit von Lösemitteln werden die Reaktionspartner in Molverhältnissen von 0,25 - 0,6 eingesetzt. Beim Einsatz von mehr als 0,6 Mol Schwefeltrioxid steigt die Viskosität der Reaktionsprodukte an, so daß praktisch nur noch in Anwesenheit inerter Lösemittel gearbeitet werden kann, um eine ausreichende Schwefeltrioxidaufnahme zu gewährleisten. Hierbei werden niedrig siedende, leicht abtrennbare Lösemittel, z.B. chlorierte Kohlenwasserstoffe, bevorzugt.

Die Sulfierung wird vorzugsweise in einem Sulfierkolben bzw. -kessel mit starkem mechanischem Rührwerk und intensiver Mantel- oder Innenkühlung durchgeführt, um eine innige Durchmischung von Gas- und Flüssigphasen, eine optimale Wärmeabfuhr und dadurch bedingte hohe Umsetzungsgrade zu gewährleisten. Die Reaktionszeit beträgt vorzugsweise zwischen 1 und 1,5 Stunden.

Gemäß einer weiteren vorteilhaften Ausführungsform der Erfindung setzt man das rohe Sulfierprodukt nach dem Abkühlen mit Nucleophilen aus der von Hydroxyl-, Alkoholat-, Phenolat- und Carboxylationen sowie von Ammoniak und primären, sekundären und tertiären Aminen gebildeten Gruppe um. Besonders bevorzugt ist die Neutralisation der sulfierten Reaktionsprodukte mit wäßrigen Basen aus der von Oxiden bzw. Hydroxiden und Carbonaten der Alkali- und Erdalkalimetalle sowie Ammoniak gebildeten Gruppe, wobei die Neutralisation mit den wäßrigen Basen in einem, bezogen auf die Schwefeltrioxidaufnahme, bis zu 30 %-igem, insbesondere bis zu 20 %-igem Überschuß erfolgt. Bevorzugte Neutralisierungsbedingungen sind 1 bis 4 Stunden bei etwa 100 °C. Weiterhin ist bevorzugt, bei der Neutralisation ein Bleichmittel zuzugeben, z.B. 1 bis 5 Gew.-% 35 %-iges Wasserstoffperoxid.

Um die bei der Umsetzung mit Nucleophilen bzw. der Neutralisation gebildeten Salze abzutrennen, reicht es normalerweise nicht aus, das Produkt nach herkömmlichen Verfahren mit Wasser auszuwaschen, da auf diese Weise eine stabile Emulsion gebildet wird, die sich mit üblichen Demulgatoren nur unvollständig auftrennen läßt. Über diese Aufarbeitungsmethode gewonnene Produkte weisen eine hohe Salzkonzentration auf und enthalten noch bis zu 50 % Wasser. Demgemäß befreit man das sulfierte, mit Nucleophilen bzw. mit wäßrigen Basen umgesetzte Reaktionsprodukt durch Zugabe von mit Wasser mischbaren, organischen Lösemitteln von unlöslichen Umsetzungsprodukten und arbeitet sie unter Entfernung der organischen Lösemittel auf. Als mit Wasser mischbare Lösemittel verwendet man vorzugsweise Alkohole aus der von $C_1$-$C_5$-Monoalkoholen, Ethylenglykol, Propylenglykol und Glycerin gebildeten Gruppe oder Ketone. Besonders bevorzugt ist hierfür der Einsatz einwertiger Alkohole mit 3 bis 4 Kohlenstoffatomen, z.B. von Propanol-1, Propanol-2, Butanol oder dergleichen, die in einer Menge von 20 bis 60, insbesondere 30 bis 50 und besonders bevorzugt von 35 bis 40 Gew.-% zugesetzt werden können. Nach Zugabe der mit Wasser mischbaren organischen Lösemittel wird das erhaltene Gemisch erwärmt; dabei dallen infolge veränderter Löslichkeiten die Neutralisationssalze vollständig aus und können z.B. durch Filtration abgetrennt werden.

Die mit Wasser mischbaren organischen Lösemittel werden anschließend im Vakuum bei ca. 100 °C abgezogen. Als Ergebnis erhält man ein Produkt in Form eines blanken, hellgelben, 100 %-igen Öls.

Das Verfahren der Erfindung wird im folgenden anhand bevorzugter Ausführungsbeispiele und Vergleichsbeispiele näher erläutert.

Beispiel 1.

a) Umsetzung von teilepoxidiertem Ricinusöl mit $SO_3$

In einem 1-1-Sulfierkolben mit Gaseinleitungsrohr, Metallflügelrührer und Mantelkühlung wurden 600 g (0,65 Mol) epoxidiertes Ricinusöl (Verseifungszahl VZ = 182,4, Iodzahl IZ = 61,0 Epoxidzahl EpZ = 0,53 %, Molgewicht aus VZ = 922,9) vorgelegt und bei 50 °C mit 78 g (0,975 Mol) $SO_3$ sulfiert.

Das $SO_3$ wurde durch Erhitzen aus einer entsprechenden Menge Oleum ausgetrieben, mit $N_2$ auf eine Konzentration von 5 Vol.-% verdünnt und innerhalb von 60 min in das Edukt eingeleitet, wobei die Temperatur des Reaktionsgemisches durch Kühlung auf Werte unter 60 °C gehalten wurde.

Nach der Sulfierung wurde das saure Reaktionsgemisch auf 10 °C abgekühlt und mit einer konzentrierten Lösung von 22 g (1,3 Mol) Ammoniak in 150 ml Wasser neutralisiert.

b) Neutralisation und Aufarbeitung des sulfierten epoxidierten Ricinusöls

Das in Stufe a erhaltene Reaktionsgemisch wurde 2 h auf dem Wasserbad bei 95-100 °C hydrolysiert. Danach wurde die Mischung bei 70 °C in 235 g (3,9 Mol) Isopropylalkohol unter Rühren gelöst.
Die dabei ausfallenden Neutralisationssalze wurden über eine Nutsche abgetrennt und das Filtrat in eine Destillationsapparatur überführt, wo die Anteile an Isopropylalkohol und wäßrigem Ammoniak bei 15 Torr/90 °C entfernt wurden.

Das erhaltene Produkt wies die folgende Kenndaten auf:
Aniontensid (Zweiphasentitrationsmethode nach Einheitsmethode DGF-H-III-10) : 0.906 mval/g
Unsulfierte Anteile (DGF-G-III-6b) : 4,5 Gew.-%
Sulfat (berechnet als Natriumsulfat) :< 0,05 Gew.-%
Chlorid : < 0,05 Gew.-%
Wasser (nach FISCHER) : 0,9 Gew.-%

Vergleichsbeispiel I.

Das in Beispiel 1, Stufe a erhaltene Reaktionsgemisch wurde 2 h auf dem Waserbad bei 95-100°C hydrolysiert. Danach wurde die Lösung mit 670 g Wasser, 300 g ges. Kochsalzlösung sowie 10 ml des Demulgators DEHYDRAN 241[R] versetzt und auf dem Dampfbad bei 95-100°C ca. 1,5 h erhitzt.

Nach Phasentrennung wurde die schwerere wäßrige Schicht abgetrennt und verworfen. Die oberen, noch einen Teil des Wassers in Form einer Emulsion enthaltende, organische Phase wurde in eine Destillationsapparatur überführt, wo das Sulfierprodukt bei 15 Torr/90°C entwässert wurde.

Das erhaltene Produkt wies die folgenden Kenndaten auf.

a) vor Destillation:

| | |
|---|---|
| Aniontensid (Zweiphasentitrationsmethode nach Einheitsmethode DGF-H-III-10) | : 0,613 mval/g |
| Unsulfierte Anteile (DGF-G-III-6b) | : 3,6 Gew.-% |
| Sulfat (berechnet als Natriumsulfat) | : 0,6 Gew.-% |
| Chlorid | : 0,2 Gew.-% |
| Wasser (nach FISCHER) | : 33 Gew.-% |

b) nach Destillation

| | |
|---|---|
| Aniontensid (Zweiphasentitrationsmethode nach Einheitsmethode DGF-H-III-10) | : 0,897 mval/g |
| Unsulfierte Anteile (DGF-G-III-6b) | : 5,5 Gew.-% |
| Sulfat (berechnet als Natriumsulfat) | : 0,7 Gew.-% |
| Chlorid | : 0,2 Gew.-% |
| Wasser (nach FISCHER) | : 2 Gew.-% |

Ein Vergleich der vorliegenden Kenndaten mit denjenigen des Produkts gemäß Beispiel 1 zeigt, daß das erfindungsgemäß erhaltene Produkt einen höheren Gehalt an Aniontensid, einen niedrigen Salzgehalt sowie einen geringeren Wassergehalt aufweist.

Vergleichsbeispiel II.

Das gemäß Beispiel 1, Stufe b erhaltene Reaktionsgemisch wurde 2 h auf dem Wasserbad bei 95-100°C hydrolysiert. Danach wurde die Lösung in eine Destillationsapparatur überführt und dort bei 15 Torr/80°C, 143 ml Wasser sowie überschüssiger, nicht in der Neutralisation umgesetzter Ammoniak abdestilliert, so daß der Restwassergehalt im Produkt ca. 5 % betrug.

Die bei der Aufkonzentrierung ausfallenden Neutralisationssalze wurden in der Folge über eine Heißdampfnutsche bei 95°C unter Verwendung eines Filterhilfsmittels abgetrennt.

Das erhaltene Produkt wies die folgenden Kenndaten auf:

| | |
|---|---|
| Aniontensid (Zweiphasentitrationsmethode nach Einheitsmethode DGF-H-III-10) | : 0,868 mval/g |
| Unsulfierte Anteile (DGF-G-III-6b) | : 5,2 Gew.-% |
| Sulfat (berechnet als Natriumsulfat) | : 1,1 Gew.-% |
| Chlorid | : < 0,05 Gew.-% |
| Wasser (nach FISCHER) | : 5 Gew.-% |

Auch hier zeigt ein Vergleich der Kenndaten, daß das erfindungsgemäß erhaltene Produkt einen höheren Aniontensidgehalt sowie niedrigere Anteile an Salz und Wasser enthält.

Vergleichsbeispiel III.

Umsetzung von epoxidiertem Ricinusöl mit Schwefelsäure

In einem 1-1-Sulfierkolben mit Metallflügelrührer und Mantelkühlung wurden 600 g (0,65 Mol) epoxidiertes Ricinusöl (Epoxidzahl 1,49 %) vorgelegt und unter intensivem Rühren portionsweise so mit 96 g (0,975 Mol) 90 %-iger Schwefelsäure versetzt, daß die Temperatur der Lösung 60°C nicht überstieg. Nach Zugabe des Sulfatierungsmittels (ca. 4 h) wurde eine weitere Stunde bei 50°C nachgerührt und das Reaktionsgemisch anschließend mit wäßriger Ammoniaklösung auf pH = 2-2,2 eingestellt, wobei Phasentrennung erfolgte.

Die wäßrige Phase, die die bei der Teilneutralisation gebildeten Salze sowie die überschüssige Schwefelsäure enthielt, wurde abgetrennt und verworfen und die zurückbleibende organische Produktphase mit konzentriertem Ammoniak auf pH = 7-7,4 eingestellt.

Das erhaltene Produkt wies die folgenden Eigenschaften auf:

| | |
|---|---|
| Aniontensid (Zweiphasentitrationsmethode nach Einheitsmethode DGF-H-III-10) | : 0,557 mval/g |
| Unsulfierte Anteile (DGF-G-III-6b) | : 21,9 Gew.-% |
| Sulfat (berechnet als Natriumsulfat) | : 1,2 Gew.-% |
| Chlorid | : 0,05 Gew.-% |
| Wasser (nach FISCHER) | : 20,2 Gew.-% |

Beim Vergleich dieses Produktes mit demjenigen des Beispiels 1 fällt insbesondere der niedrige Aniontensidgehalt, der hohe Gehalt an unsulfierten Anteilen sowie der hohe Wassergehalt des nicht erfindungsgemäß erhaltenen Produkts auf.

Beispiel 2.

Umsetzung von epoxidiertem Ricinusöl mit Schwefeltrioxid in 1,2-Dichlorethan

53 g (0,66 Mol) $SO_3$, das auf dem beschriebenen Weg gewonnen werden kann, wurde in 50 ml 1,2-Dichlorethan eingeleitet und bei 0°C im organischen Substrat gelöst.

In einem 500-ml-Dreihalskolben mit Metallflügelrührer, Tropftrichter und Innenthermometer wurden 300 g (0,32 Mol) eines epoxidierten Ricinusöls (Epoxidzahl 0,53) vorgelegt und in ca. 250 ml vorgetrocknetem 1,2-Dichlorethan gelöst.

Unter intensivem Rühren wurde die vorbereitete Lösung des $SO_3$'s im organischen Solvens so zugetropft, daß die Temperatur der Lösung nicht über 20°C anstieg (Zutropfzeit ca. 60 min). Anschließend wurde das Lösungsmittel sehr rasch bei 30°C im Hochvakuum abgezogen, der saure Ester mit 13,5 g (0,8 Mol) $NH_3$ in 120 ml Wasser neutralisiert und wie in Beispiel 1, Stufe b beschrieben, aufgearbeitet.

Das erhaltene Produkt wies die folgenden Kenndaten auf:

Aniontensid (Zweiphasentitrationsmethode nach Einheitsmethode DGF-H-III-10) : 0,88 mval/g

Unsulfierte Anteile (DGF-G-III-6b) : 14,5 Gew.-%

Sulfat (berechnet als Natriumsulfat) : 1,8 Gew.-%

Chlorid : < 0,05 Gew.-%

Wasser (nach FISCHER) : 0,9 Gew.-%

Beispiel 3.

Umsetzung von epoxidiertem Ricinolsäuremonoglycerid mit Schwefeltrioxid

In einem 1-1-Sulfierkolben mit Gaseinleitungsrohr, Metallflügelrührer und Mantelkühlung wurden 335 g (1,00 Mol) epoxidiertes Ricinolsäuremonoglycerid (Verseifungszahl VZ = 167,5, Epoxidzahl EpoZ = 1,49 %, Molgewicht aus VZ = 334,9) vorgelegt und bei 50°C mit 40 g (0,35 Mol) $SO_3$ sulfiert.

Das $SO_3$ wurde wie in Beispiel 1, Stufe a beschrieben, gewonnen und innerhalb von 35 min in das Edukt eingeleitet, wobei die Temperatur des Reaktionsgemisches durch Kühlung unter 60°C gehalten wurde.

Nach der Sulfierung wurde das saure Reaktionsgemisch auf 10°C abgekühlt, mit einer konzentrierten Lösung von 9 g (0,48 Mol) $NH_3$ in 75 ml Wasser neutralisiert und dann wie in Beispiel 1, Stufe b beschrieben, aufgearbeitet.

Das erhaltene Produkt wies die folgenden Kenndaten auf:

Aniontensid (Zweiphasentitrationsmethode nach Einheitsmethode DGF-H-III-10) : 2,65 mval/g

Unsulfierte Anteile (DGF-G-III-6b) : 9,8 Gew.-%

Sulfat (berechnet als Natriumsulfat) : 0,4 Gew.-%

Chlorid : < 0,05 Gew.-%

Wasser (nach FISCHER) : 0,9 Gew.-%

## Patentansprüche

1. Verfahren zur Herstellung von Umsetzungsprodukten epoxidierter Ricinolsäureglyceride mit Schwefeltrioxid, dadurch gekennzeichnet, daß man die epoxidierten Ricinolsäureglyceride mit einem trockenen, mit einem Trägergas verdünnten Strom gasförmigen Schwefeltrioxids zur Reaktion bringt und das sulfierte Reaktionsprodukt mit Nucleophilen umsetzt.

2. Verfahren nach Anspruch 1, dadurch gekennzeichnet, daß man Luft oder Stickstoff als Trägergas verwendet.

3. Verfahren nach einem der Ansprüche 1 bis 2, dadurch gekennzeichnet, daß man einen Trägergasstrom mit einem Schwefeltrioxidgehalt von 2 bis 10, insbesondere 5 bis 8 Vol.-% verwendet.

4. Verfahren nach einem der Ansprüche 1 bis 3, dadurch gekennzeichnet, daß man die epoxidierten Ricinolsäureglyceride bei Temperaturen von 30 bis 80°C mit dem Schwefeltrioxid umsetzt.

5. Verfahren nach einem der Ansprüche 1 bis 4, dadurch gekennzeichnet, daß man die epoxidierten Ricinolsäureglyceride bei Temperaturen von 40 bis 50°C mit dem Schwefeltrioxid umsetzt.

6. Verfahren nach einem der Ansprüche 1 bis 5, dadurch gekennzeichnet, daß man pro Mol vorhandenem Ricinolsäurerest mindestens 0,25 - 1 Mol Schwefeltrioxid einsetzt.

7. Verfahren nach einem der Ansprüche 1 bis 6, dadurch gekennzeichnet, daß man in Abwesenheit von Lösemitteln pro Mol Ricinolsäurerest 0,25 bis 0,6 Mol Schwefeltrioxid einsetzt.

8. Verfahren nach einem der Ansprüche 1 bis 7, dadurch gekennzeichnet, daß man in Gegenwart inerter Lösemittel pro Mol Ricinolsäurerest 0,6 bis 1,0 Mol Schwefeltrioxid einsetzt.

9. Verfahren nach einem der Ansprüche 1 bis 8, dadurch gekennzeichnet, daß man die Umsetzung mit Schwefeltrioxid innerhalb von 1 bis 1,5 h durchführt.

10. Verfahren nach einem der Ansprüche 1 bis 9, dadurch gekennzeichnet, daß man Nucleophile aus der von Hydroxyl-, Alkoholat-, Phenolat- und Carboxylationen sowie von Ammoniak und primären, sekundären und tertiären Aminen gebildeten Gruppe verwendet.

11. Verfahren nach einem der Ansprüche 1 bis 10, dadurch gekennzeichnet, daß man die sulfierten Reaktionsprodukte mit wäßrigen Basen aus der von Oxiden bzw. Hydroxiden und Carbonaten der Alkali- und Erdalkalimetalle sowie Ammoniak gebildeten Gruppe neutralisiert.

12. Verfahren nach einem der Ansprüche 1 bis 11, dadurch gekennzeichnet, daß man die sulfierten Reaktionsprodukte mit den wäßrigen Basen in einem bis zu 30 %-igem, insbesondere bis zu 20 %-igem Überschuß neutralisiert.

13. Verfahren nach einem der Ansprüche 1 bis 12, dadurch gekennzeichnet, daß man während der Neutralisation ein Bleichmittel zugibt.

14. Verfahren nach einem der Ansprüche 1 bis 13, dadurch gekennzeichnet, daß man das sulfierte, mit Nucleophilen umgesetzte Reaktionsprodukt durch Zugabe von mit Wasser mischbaren, organischen Lösemitteln von unlöslichen Umsetzungsprodukten befreit sowie unter Entfernung der organischen Lösemittel aufarbeitet.

15. Verfahren nach einem der Ansprüche 1 bis 14, dadurch gekennzeichnet, daß man als mit Wasser mischbare Lösemittel Alkohole aus der von $C_1$ bis $C_5$-Monoalkoholen, Ethylenglykol, Propylenglykol, Glycerin und Ketonen gebildeten Gruppe verwendet.

16. Verfahren nach einem der Ansprüche 1 bis 15, dadurch gekennzeichnet, daß man als mit Wasser mischbare organische Lösemittel einwertige Alkohole mit 3 bis 4 Kohlenstoffatomen verwendet.

17. Verfahren nach einem der Ansprüche 1 bis 16, dadurch gekennzeichnet, daß man die mit Wasser mischbaren organischen Lösemittel in einer Menge von 20 bis 60, insbesondere 30 bis 50 Gew.-% zusetzt.

18. Verfahren nach einem der Ansprüche 1 bis 17, dadurch gekennzeichnet, daß man teilepoxidierte Ricinolsäureglyceride sulfiert, bei denen 10 bis 30, insbesondere 10 bis 15 % der ursprünglich vorhandenen olefinischen Doppelbindungen epoxidiert sind.

## Claims

1. A process for the preparation of reaction products of epoxidized ricinoleic acid glycerides with sulfur trioxide, characterized in that the epoxidized ricinoleic acid glycerides are reacted with a dry, gaseous sulfur trioxide diluted with a carrier gas and the sulfonated reaction product is reacted with nucleophiles.

2. A process as claimed in claim 1, characterized in that air or nitrogen is used as the carrier gas.

3. A process as claimed in claim 1 or 2, characterized in that a carrier gas stream having a sulfur dioxide content of 2 to 10% by volume and more especially 5 to 8% by volume is used.

4. A process as claimed in any of claims 1 to 3, characterized in that the epoxidized ricinoleic acid glycerides are reacted with the sulfur trioxide at temperatures of 30 to 80 °C.

5. A process as claimed in any of claims 1 to 4, characterized in that the epoxidized ricinoleic acid glycerides are reacted with the sulfur trioxide at temperatures of 40 to 50 °C

6. A process as claimed in any of claims 1 to 5, characterized in that at least 0.25 to 1 mol sulfur trioxide is used per mol ricinoleic acid residue present.

7. A process as claimed in any of claims 1 to 6, characterized in that, in the absence of solvents, 0.25 to 0.6 mol sulfur trioxide is used per mol ricinoleic acid residue.

8. A process as claimed in any of claims 1 to 7, characterized in that, in the presence of inert solvents, 0.6 to 1.0 mol sulfur trioxide is used per mol ricinoleic acid residue.

9. A process as claimed in any of claims 1 to 8, characterized in that the reaction with sulfur trioxide is carried out in 1 to 1.5 h.

10. A process as claimed in any of claims 1 to 9, characterized in that nucleophiles from the group consisting of hydroxyl, alcoholate, phenolate and carboxylate ions and of ammonia and primary, secondary and tertiary amines are used.

**11.** A process as claimed in any of claims 1 to 10, characterized in that the sulfonated reaction products are neutralized with aqueous bases from the group consisting of oxides or hydroxides and carbonates of the alkali and alkaline earth metals and ammonia.

**12.** A process as claimed in any of claims 1 to 11, characterized in that the sulfonated reaction products are neutralized with the aqueous bases in an excess of up to 30% and more especially up to 20%.

**13.** A process as claimed in any of claims 1 to 12, characterized in that a bleach is added during the neutralization.

**14.** A process as claimed in any of claims 1 to 13, characterized in that the sulfonated reaction product reacted with nucleophiles is freed from insoluble reaction products by addition of water-miscible organic solvents and is worked up with removal of the organic solvents.

**15.** A process as claimed in any of claims 1 to 14, characterized in that alcohols from the group consisting of $C_1$-$C_5$ monoalcohols, ethylene glycol, propylene glycol, glycerol and ketones are used as the water-miscible solvents.

**16.** A process as claimed in any of claims 1 to 15, characterized in that monohydric alcohols containing 3 to 4 carbon atoms are used as the water-miscible organic solvents.

**17.** A process as claimed in any of claims 1 to 16, characterized in that the water-miscible organic solvent is added in a quantity of 20 to 60% by weight and more especially in a quantity of 30 to 50% by weight.

**18.** A process as claimed in any of claims 1 to 17, characterized in that partially epoxidized ricinoleic acid glycerides, in which 10 to 30% and more especially 10 to 15% of the olefinic double bonds originally present are epoxidized, are sulfonated.

**Revendications**

**1.** Procédé pour la préparation de produits de transformation de glycérides époxydés de l'acide ricinoléique avec du trioxyde de soufre, **caractérisé en ce qu'**on fait réagir les glycérides époxydés de l'acide ricinoléique avec un courant sec de trioxyde de soufre gazeux, dilué avec un gaz de support et on transforme le produit réactionnel sulfoné avec des composés nucléophiles.

**2.** Procédé selon la revendication 1, **caractérisé en ce que,** comme gaz de support, on utilise de l'air ou de l'azote.

**3.** Procédé selon l'une quelconque des revendications 1 ou 2, **caractérisé en ce qu'**on utilise un courant de gaz de support ayant une teneur en trioxyde de soufre de 2 à 10, en particulier de 5 à 8 pour cent en volume.

**4.** Procédé selon l'une quelconque des revendications 1 à 3, **caractérisé en ce qu'**on fait réagir les glycérides époxydés de l'acide ricinoléique à des températures de 30 à 80° C, avec le trioxyde de soufre.

**5.** Procédé selon l'une quelconque des revendications 1 à 4, **caractérisé en ce qu'**on fait réagir les glycérides époxydés de l'acide ricinoléique à des températures de 40 à 50° C, avec le trioxyde de soufre.

**6.** Procédé selon l'une quelconque des revendications 1 à 5, **caractérisé en ce que,** par mole de radical de l'acide ricinoléique présent, on met en oeuvre au moins de 0,25 à 1 mole de trioxyde de soufre.

**7.** Procédé selon l'une quelconque des revendications 1 à 6, **caractérisé en ce que,** en l'absence de solvants, par mole de radical d'acide ricinoléique, on met en oeuvre de 0,25 à 0,6 mole de trioxyde de soufre.

**8.** Procédé selon l'une quelconque des revendications 1 à 7, **caractérisé en ce que,** en présence de solvants inertes, par mole de radical de l'acide ricinoléique, on met en oeuvre de 0,6 à 1,0 mole de trioxyde de soufre.

**9.** Procédé selon l'une quelconque des revendications 1 à 8, **caractérisé en ce qu'**on effectue la mise en réaction avec le trioxyde de soufre dans un laps de temps s'étalant entre 1 et 1,5 heure.

**10.** Procédé selon l'une quelconque des revendications 1 à 9, **caractérisé en ce qu'**on utilise des composés nucléophiles du groupe formé par des ions hydroxyle, alcoolate, phénolate et carboxylate, ainsi que par l'ammoniac et des amines primaires, secondaires et tertiaires.

**11.** Procédé selon l'une quelconque des revendications 1 à 10, **caractérisé en ce qu'**on neutralise les produits réactionnels sulfonés avec des bases aqueuses du groupe formé par des oxydes ou des hydroxydes et des carbonates de métaux alcalins et alcalino-terreux, ainsi que par l'ammoniac.

12. Procédé selon l'une quelconque des revendications 1 à 11, **caractérisé en ce qu'**on neutralise les produits réactionnels sulfonés avec les bases aqueuses en un excès allant jusqu'à 30%, en particulier jusqu'à 20%.

13. Procédé selon l'une quelconque des revendications 1 à 12, **caractérisé en ce que,** pendant la neutralisation, on ajoute un décolorant.

14. Procédé selon l'une quelconque des revendications 1 à 13, **caractérisé en ce qu'**on libère le produit réactionnel sulfoné mis à réagir avec des composés nucléophiles, des produits de transformation insolubles, par addition de solvants organiques miscibles à l'eau et on le traite ultérieurement en éliminant les solvants organiques.

15. Procédé selon l'une quelconque des revendications 1 à 14, **caractérisé en ce que,** comme solvants miscibles à l'eau, on utilise des alcools du groupe formé par des monoalcools en $C_1$-$C_5$, l'éthylèneglycol, le propylèneglycol, la glycérine et les cétones.

16. Procédé selon l'une quelconque des revendications 1 à 15**, caractérisé en ce que,** comme solvants organiques miscibles à l'eau, on utilise des alcools monovalents contenant de 3 à 4 atomes de carbone.

17. Procédé selon l'une quelconque des revendications 1 à 16, **caractérisé en ce qu'**on ajoute les solvants organiques miscibles à l'eau en une quantité de 20 à 60, en particulier de 30 à 50 pour cent en poids.

18. Procédé selon l'une quelconque des revendications 1 à 17, **caractérisé en ce qu'**on soumet à une sulfonation, des glycérides de l'acide ricinoléique partiellement époxydés, dans lesquels de 10 à 30, en particulier de 10 à 15% des doubles liaisons oléfiniques présentes à l'origine sont époxydées.